# EUROPEAN PATENT APPLICATION

(11) **EP 2 270 201 A1**
(43) Date of publication of application: **05.01.2011**
(21) Application number: 09172185.2
(22) Date of filing: 05.10.2009
(51) Int. Cl.: C12Q 1/48, G01N 33/50

(54) **Detection of unhealthy cell and uses thereof**

(30) Priority: 01.07.2009 US 495967
(71) Applicant: National Tsing Hua University, Hsinchu 30013 (TW)
(72) Inventor: YANG, Shiaw-Der, 30013, Hsinchu (TW)
(74) Representative: Schwerbrock, Florian

(57) **Abstract**

Provided herein is a method for detecting the presence of unhealthy cell in a subject using the expression of PDPK F_{A}/GSK-3α in cell and uses thereof. In another aspect, provided herein is a method for predicting if a subject is systemically unhealthy and at risk of developing systemic diseases and syndromes.

## Description

**Technical Field**

The present invention relates to methods and kits useful in identifying and detecting an unhealthy cell. The present invention further provides methods and kits useful in predicting if a subject is systemically unhealthy and at risk of developing systemic diseases and syndromes by using proline-directed protein kinase F_{A}/glycogen synthase kinase 3 alpha (PDPK F_{A}/GSK-3α) as a diagnostic indicator.

**Background Art**

It is widely accepted that the bone marrow is a major source of both hematopoietic stem/progenitor cells and mesenchymal stem/progenitor cells and their derivatives recruited and homing to the injured tissue ensuring hematopoietic and skeletal homeostasis. It is now clear that the bone marrow contributes to systemic tissue homeostasis from wound healing to end-stage fibrotic disease and neoplasia. The intrinsic defect in bone marrow stem and progenitor cells and their derivatives such as stromal and inflammatory cells may represent the major cause for aberrant wound healing, chronic inflammation, systemic diseases and syndromes such as systemic fibroses, infections, obstructions, emboli and multiple organ failures. However, the controversial and paradoxical roles of bone marrow-derived cells (BMDCs) among normal healing wounds, nonhealing wounds such as systemic ulcer and particularly overhealing wounds such as systemic fibrosis and neoplasia remain a great challenge to solve the current bone marrow-related systemic diseases, syndromes and transplantation problems (Bingle et al, J Pathol, 2002, 196:254-265; De Wever and Mareel, J Pathol, 2003, 200:429-447; Dunsmore and Shapiro, J Clin Invest, 2004, 113:180-182; Condeelis and Pollard, Cell, 2006, 124: 263-266; Yin and Li, J Clin Invest, 2006, 116:1195-1201; Karnoub et al, Nature, 2007, 449:557-563; Biswas et al, J Immunol, 2008, 180: 2011-2017; Kisseleva and Brenner, Exp Biol Med, 2008, 233:109-122; Lin et al, Cells Tissues Organs, 2008, 188:178-188; Schafer and Werner, Nat Rev Mol Cell Biol, 2008, 9:628-638; Valtieri and Sorrentino, J Cell Physiol, 2008, 217:296-300; Wynn, J Pathol, 2008, 214:199-210; Mishra et al, Cancer Res, 2009, 69:1255-1258).

Proline-directed protein kinase F_{A} (PDPK F_{A})/glycogen synthase kinase 3α (GSK-3α) was originally identified as a specific protein phosphatase activating factor A (Vandenheede et al, J Biol Chem, 1980, 255: 11768-11774; Yang et al, J Biol Chem, 1980, 255:11759-11767; Woodgett, EMBO J, 1990, 9:2431-2438). In this lab, PDPK F_{A}/GSK-3α was further characterized as a multisubstrate/multifunctional PDPK essential for the development of anti-apoptosis, tumorigenesis, invasion and chemoresistance of various types of cancer cells (Hsu et al, Br J Cancer, 2000, 82:1480-1484; Hsu et al, Cancer, 2000, 89:1004-1011; Yang et al, Clin Cancer Res, 2000, 6:1024-1030; Hsu et al, Cancer, 2001, 92:1753-1758; Hsu et al, Int J Cancer, 2001, 91:650-653; Chung et al, Cancer, 2002, 95:1840-1847; Hsueh et al, Cancer, 2002, 95:775-783; Fu and Yang, Anticancer Res, 2004, 24:1489-1494). However, the systemic role of this signaling molecule particularly in bone marrow-derived cells remains largely unknown and needs to be established.

It has been appreciated that a predictive relationship can exist between the presence of one single unique molecule expression in a cell and the health status of a subject regardless of the etiology of the beyond curable disease. Developing universally applicable molecule expression in a cell for determining a subject's risk of developing a beyond curable disease has never been accomplished.

**Disclosure of the Invention**

The unhealthy cell provided herein represents a universally, applicable predictor useful for very early detection of systemic deregulation and imbalance of hematopoiesis, hemastasis, homeostasis, and immune system in association with systemic fibroses, immune suppression, infections, obstructions, emboli and multiple organ failures originated from intrinsic defect in bone marrow. More particularly, the unhealthy cell is a reliable predictor for determining if a subject is systemically unhealthy and at risk of developing beyond curable systemic disease regardless of its etiological origin.

Thus, in one aspect, provided herein is a method for detecting the presence of a cellular expression profile preferably a bone marrow-derived cell in a subject indicative of unhealthy cell, which method comprises obtaining a biological sample from said subject; determining the expression of PDPK F_{A}/GSK-3α in cell in said sample wherein an increased expression level of PDPK F_{A}/GSK-3α in cell of said subject indicates the presence of unhealthy cell. In some embodiments, the expression of PDPK F_{A}/GSK-3α is determined by assaying PDPK F_{A} /GSK-3α protein levels such as an immunoassay using antibodies specific for PDPK F_{A}/GSK-3α. In other aspects, the expression can be determined by assessing activity, protein, mRNA or DNA level. The biological sample can be bone marrow, cord blood, peripheral blood, tissue sample, ascites, pleural effusions or body fluids.

In one aspect, identification of unhealthy cell in a biological sample is useful for predicting if a subject is systemically unhealthy and at risk of developing systemic diseases and syndromes.

In another aspect, provided herein is a diagnostic kit for determining the presence of unhealthy cell in a biological sample comprising at least one reagent for determining the expression of PDPK F_{A}/GSK-3α in said sample, and printed instructions for assessing the presence of unhealthy cell, packaged together in a container. Further detection reagents may also be included.

Furthermore, in another aspect, provided herein is a method for predicting if a subject is systemically unhealthy and at risk of developing systemic diseases and syndromes, which method comprises obtaining a biological sample from said subject; determining the expression of PDPK F_{A}/GSK-3α in said sample wherein an increased expression level of PDPK F_{A}/GSK-3α in comparison to a normal cell in said subject predicts if a subject is systemically unhealthy and at risk of developing systemic diseases and syndromes. In some embodiments, the expression of PDPK F_{A}/GSK-3α is determined by assaying PDPK F_{A}/GSK-3α protein levels such as an immunoassay using antibodies specific for PDPK F_{A}/GSK-3α. In other aspects, the expression can be determined by assessing activity, protein, mRNA or DNA level. The biological sample can be bone marrow, cord blood, peripheral blood, tissue sample, ascites, pleural effusions or body fluids.

If necessary, the cell can be isolated for detection of unhealthy cell highly expressing PDPK F_{A}/GSK-3α by specific magnetic beads or flow cell sorter essentially as described by Moioli et al (PLoS ONE, 3:e3922, 2008).

These and other objects and features of the invention will become more fully apparent when the following detailed description is read in conjunction with the accompanying figures and examples.

**Brief Description of the Drawings**

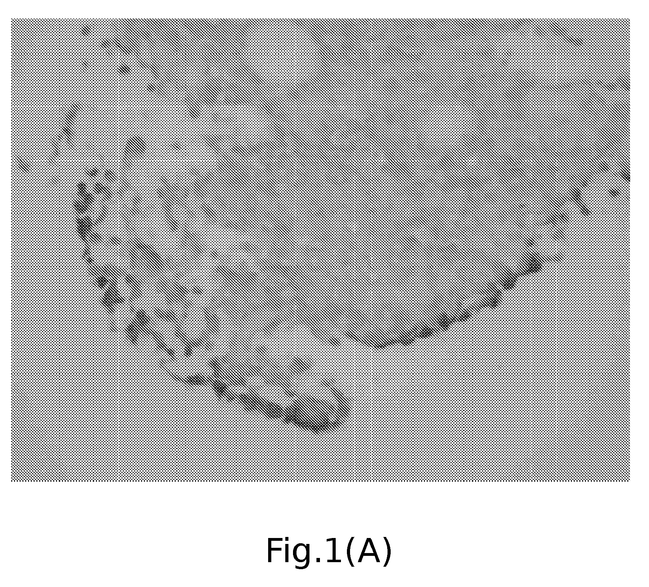

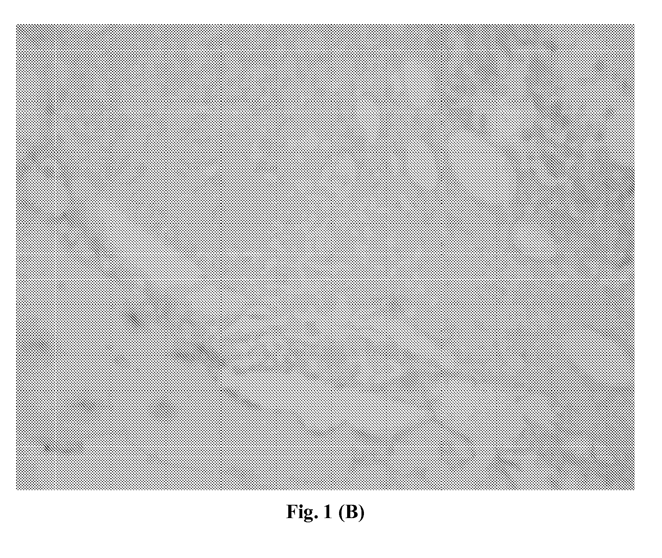

Fig. 1 depicts the aberrant expression patterns of PDPK F_{A}/GSK-3α in bone marrow-derived cells (BMDC) by co-immunohistochemical staining of a rare population of CD34⁺and CD34⁻ stem/progenitor cells associated with very strong expressions (>3+) of PDPK F_{A}/GSK-3α and their possible derivatives with moderate-strong expressions (2+ to 3+) of PDPK F_{A} /GSK-3α in bone marrow of leukemia patients with progressive diseases. (A). Positive pattern costaining with CD34, (B). Negative pattern costaining with CD34.

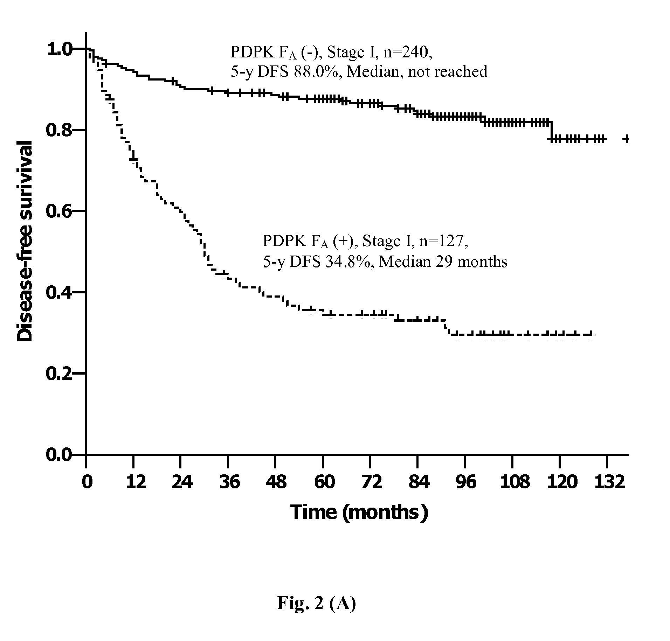

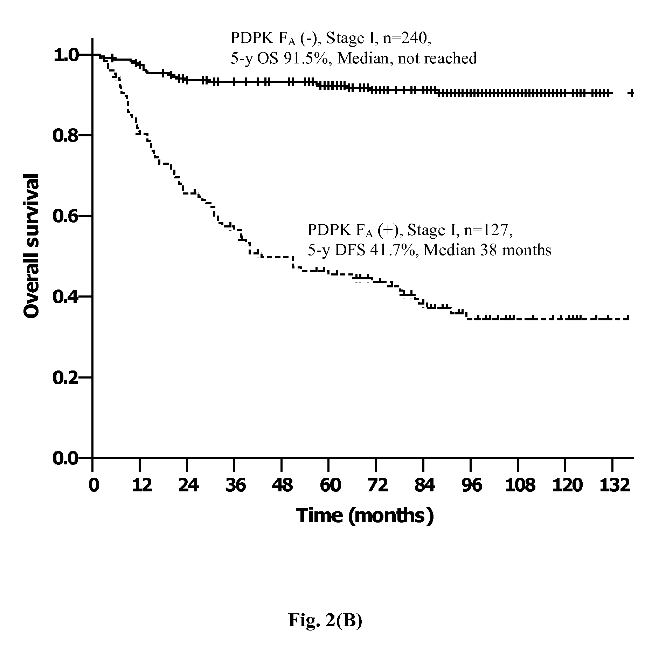

Fig. 2 depicts the Kaplan-Meier disease-free survival (DFS) and overall survival (OS) curve of various types of patients with very early stage I tumors shown in Table 1 with respect to the presence and absence of aberrant expressions of PDPK F_{A}/GSK-3α within stroma tissues of breast, bile duct, colorectum, cervix, esophagus, stomach, liver, lung, oral cavity, ovary, pancreas, prostate and kidney in the recruited BMDC. (A). disease-free survival (DFS), (B). overall survival (OS).

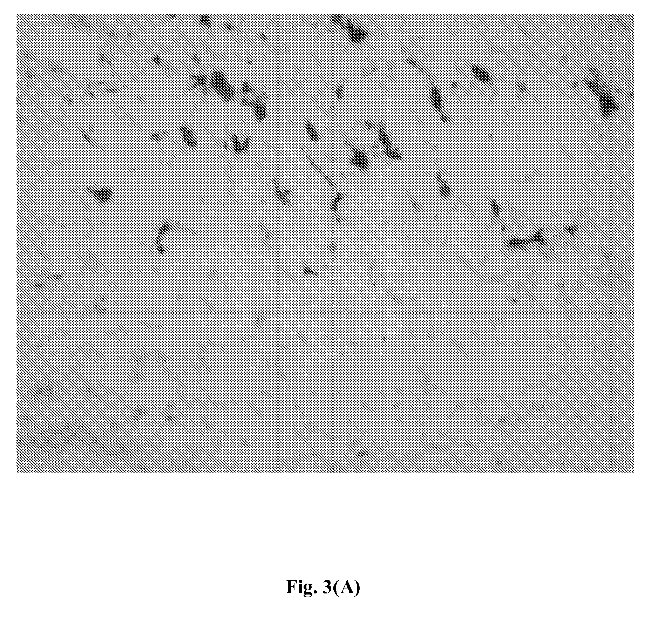

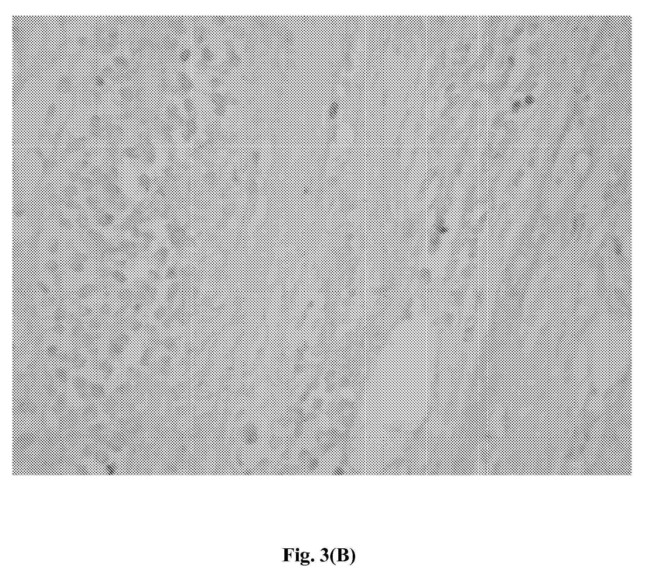

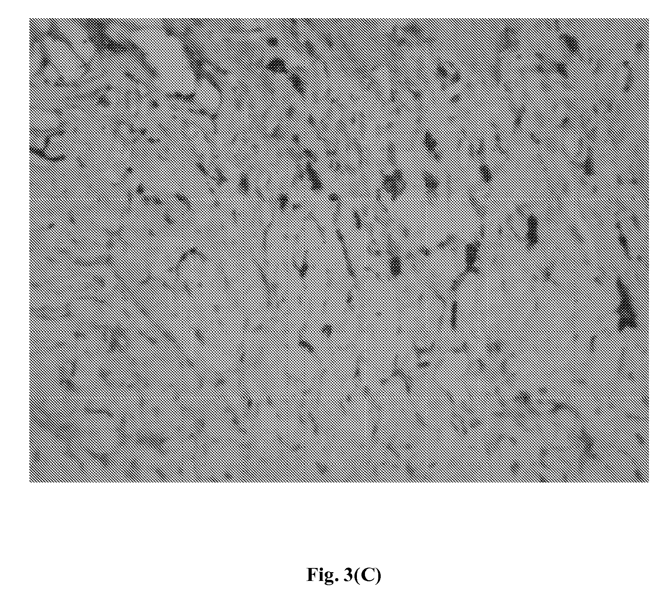

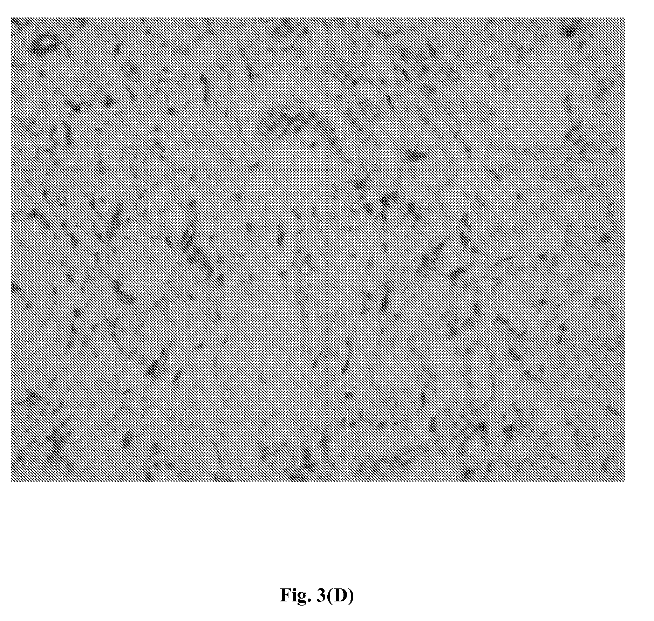

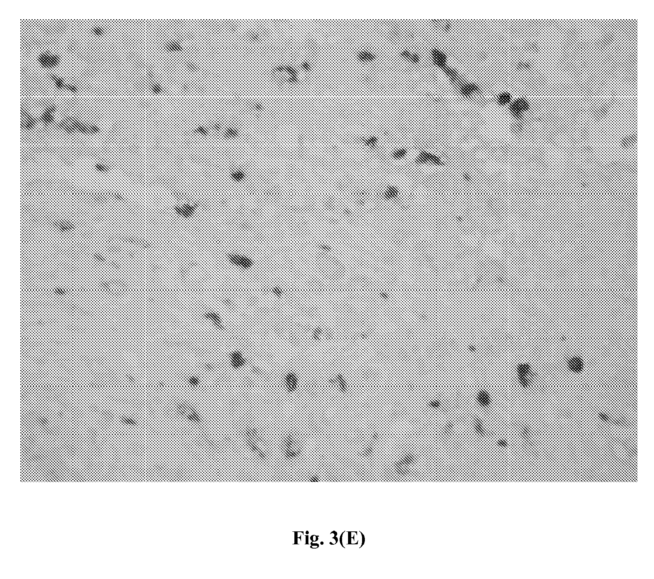

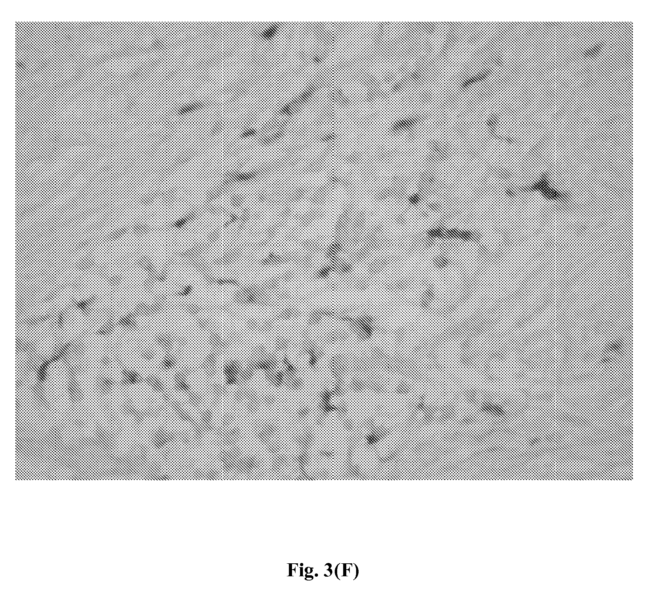

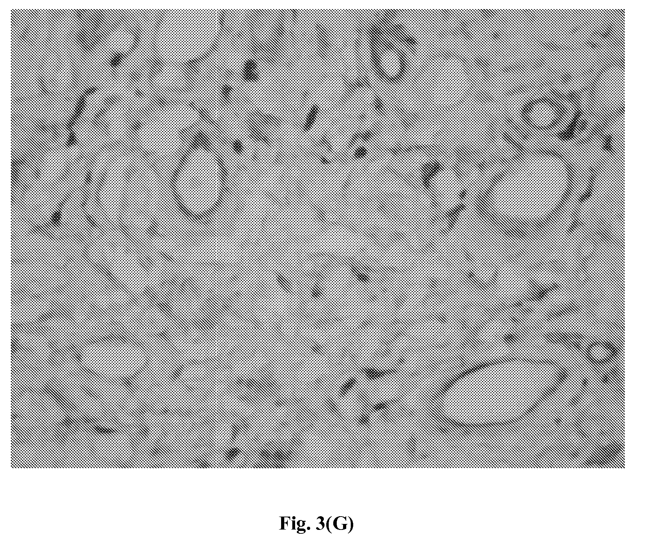

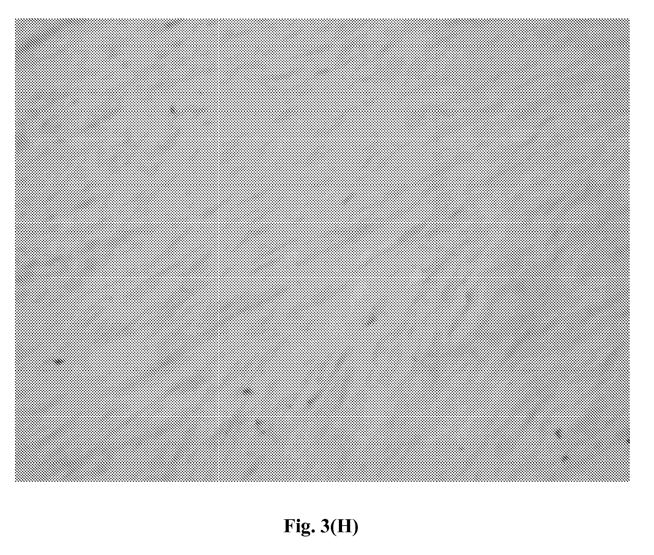

Fig. 3 depicts the representative aberrant expression patterns of PDPK F_{A} /GSK-3α in stage I tumor stromas by co-immunohistochemical staining of a rare population of CD34⁺/vimentin-, CD34⁺/vimentin+and CD34-/vimentin + cells associated with very strong expression (>3+) of PDPK F_{A}/GSK-3α and a relatively large population of their derivatives vimentin⁺ fibroblasts/mesenchymal cells, CD68⁺ macrophages and a-SMA⁺ myofibroblast associated with moderate to strong expressions (2+ to 3+) of PDPK F_{A}/GSK-3α. (A). Positive pattern costaining with CD34, (B). Negative pattern costaining with CD34, (C). Positive pattern costaining with vimentin, (D). Negative pattern costaining with vimentin, (E). Positive pattern costaining with CD68, (F). Negative pattern costaining with CD68, (G). Positive pattern costaining with α-SMA, (H). Negative pattern costaining with α-SMA.

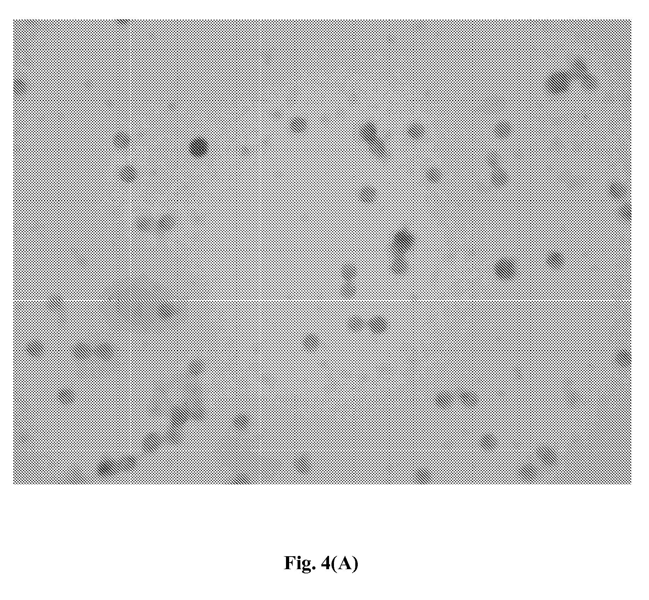

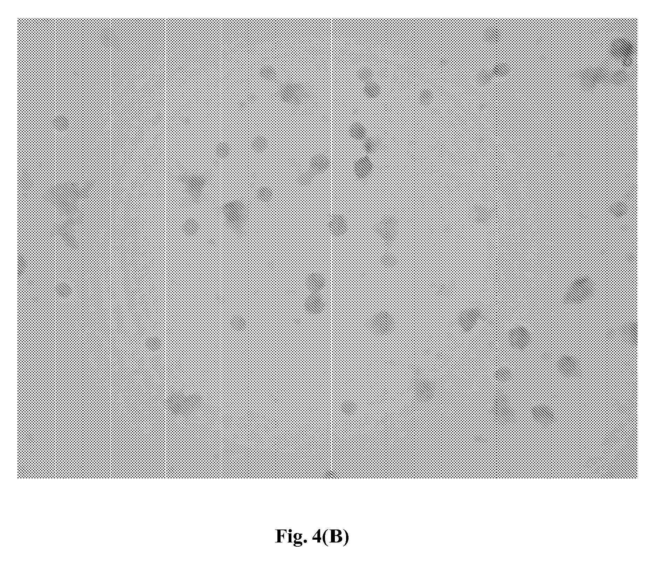

Fig. 4 establishes PDPK F_{A}/GSK-3α as an early unhealthy signal by co-immunocytochemical staining of CD34⁺ fibrocytes-like cells associated with strong expression of PDPK F_{A}/GSK-3α in peripheral blood of children from cancer family or suffering from repeated cycles of inflammations. (A). Positive pattern, (B). Negative pattern.

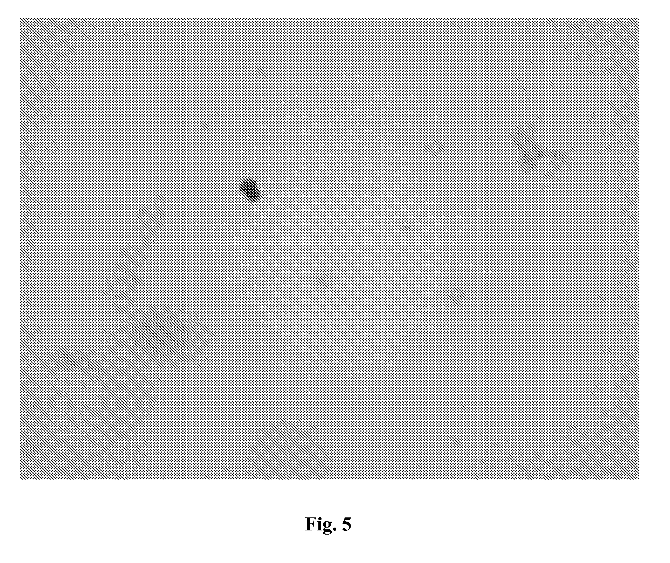

Fig. 5 further establishes PDPK F_{A}/GSK-3α as a very early unhealthy signal by co-immunocytochemical staining of CD34⁺ hematopoietic stem/progenitor cells associated with strong expression of PDPK F_{A} /GSK-3α in umbilical cord blood of systemically unhealthy child suffering from immune system disorder characteristic of Kawasaki syndrome.

**Modes of Carrying Out the Invention**

For clarity of disclosure, and not by way of limitation, the detailed description of the invention is divided into the subsections that follow.

Definition

Unless defined otherwise, all technical and scientific terms used herein have the same meaning as is commonly understood by one of ordinary skill in the art to which this invention belongs. All patents, applications, published applications and other publications, and Genbank Accession numbers referred to herein are incorporated by reference in their entirety. If a definition set forth in this section is contrary to or otherwise inconsistent with a definition set forth in the patents, applications, published applications and other publications that are herein incorporated by reference, the definition set forth in this section prevails over the definition that is incorporated herein by reference.

As used herein, "a" or "an" means "at least one" or "one or more."

As used herein, the term "PDPK F_{A}/GSK-3α" refers to the multisubstrate/multifunctional proline-directed protein kinase F_{A} also known as glycogen synthase kinase-3alpha (Woodgett, EMBO J, 1990, 9: 2431-2438; Yang, Curr Cancer Drug Targets, 2004, 4:591-596). The Genbank Accession numbers for this protein are AAD11986 and AAH27984.

As used herein, "biological sample" refers to any sample from a biologic source, including but not limited to bone marrow, blood, tissue sample, ascites, pleural effusions or body fluids.

As used herein, the term "antibody" refers to an isolated or recombinant binding agent that comprises the necessary variable region sequences to specifically bind an antigenic epitope. Therefore, an antibody is any form of antibody or fragment thereof that exhibits the desired biological activity, e.g., binding the specific target antigen. Thus, it is used in the broadest sense and specifically covers monoclonal antibodies (including full length monoclonal antibodies), polyclonal antibodies, human antibodies, humanized antibodies, chimeric antibodies, nanobodies, diabodies, multispecific antibodies (e.g., bispecific antibodies), and antibody fragments including but not limited to scFv, Fab, and Fab2, so long as they exhibit the desired biological activity, e.g., specifically bind PDPK F_{A} /GSK-3α.

As used herein, the term "subject" refers to any living organism, preferably the subjects are mammals. Exemplary subjects include, but are not limited to humans.

As used herein, the term "unhealthy cell" refers to a cell preferably a BMDC associated with aberrant expression of PDPK F_{A}/GSK-3α.

Unless otherwise indicated, all terms used herein have the same meaning as they would to one skilled in the art and the practice of this invention will be employed, conventional techniques of biochemical and clinical pathological technology, which are within the knowledge of those of skill of the art.

**Methods and kits for detecting unhealthy cells**

A cell preferably a BMDC associated with aberrant expression of PDPK F_{A} /GSK-3α, the unhealthy cell, offers a tool to identify if a subject is systemically unhealthy and at risk of developing systemic diseases and syndromes. In one aspect, identification of an unhealthy cell in a biological sample is useful for predicting the development of systemic diseases and syndromes.

Thus, in one aspect, provided herein is a method for detecting the presence of a cellular expression profile in a subject indicative of unhealthy cell, which method comprises obtaining a biological sample from said subject; determining the expression of PDPK F_{A}/GSK-3α in cell in said sample wherein an increased expression level of PDPK F_{A}/GSK-3α in cell of said subject indicates the presence of unhealthy cell. In some embodiments, the expression of PDPK F_{A}/GSK-3α is determined by assaying PDPK F_{A}/GSK-3α protein level such as an immunoassay using antibodies specific for PDPK F_{A}/GSK-3α. In other aspects, the expression can be determined by assessing activity, protein, RNA or DNA level. The biological sample can be bone marrow, cord blood, peripheral blood, tissue sample, ascites, pleural effusions or body fluids.

In another aspect, provided herein is a kit for determining the presence of unhealthy cell in a biological sample comprising at least one reagent for determining the expression of PDPK F_{A}/GSK-3α in a cell in said sample, and printed instructions for assessing the relative levels of PDPK F_{A} /GSK-3α, packaged together in a container.

Any suitable means of detecting PDPK F_{A}/GSK-3α expression may be employed. The expression can be determined by assessing activity, protein, RNA or DNA levels in cell from a biological sample. For example, an immunoassay using an antibody specific for PDPK F_{A}/GSK-3α may be employed. Suitable means include, but are not limited to immunohistochemical analysis, immunocytochemical analysis, flow cytometry analysis, Western blot analysis, Northern blot analysis, RT-PCR and phosphorylation assays on specific substrates. With immunehistochemical staining techniques, a cell sample is prepared, typically by dehydration and fixation, followed by reaction with labeled antibodies specific for the gene product coupled, where the labels are usually visually detectable, such as enzymatic labels, florescent labels, luminescent labels, and the like.

According to one embodiment, tissue samples are obtained from subjects and the samples are embedded then cut to e.g. 3-5 µm, fixed, mounted and dried according to conventional tissue mounting techniques. The fixing agent may comprise formalin. The embedding agent for mounting the specimen may comprise, e.g., paraffin. The samples may be stored in this condition. Following deparaffinization and rehydration, the samples are contacted with an immunoreagent comprising an antibody specific for PDPK F_{A}/GSK-3α. The antibody may comprise a polyclonal or monoclonal antibody. The antibody may comprise an intact antibody, or fragments thereof capable of specifically binding PDPK F_{A}/GSK-3α protein. Appropriate polyclonal antisera or other antibody may be prepared by immunizing appropriate host animals with PDPK F_{A}/GSK-3α protein, or a suitable fragment thereof, and collecting and purifying the antisera according to conventional techniques known to those skilled in the art. Monoclonal or polyclonal antibodies, preferably monoclonal, specifically reacting with PDPK F_{A}/GSK-3α, may be made by methods well known in the art. See, e.g., Harlow and Lane (1988) Antibodies: A Laboratory Manual, Cold Spring Harbor Laboratories; Goding (1986) Monoclonal Antibodies: Principles and Practice, 2d ed., Academic Press, New York. Also, recombinant antibodies may be produced by methods known in the art, including but not limited to, the methods described in U.S. Pat. No. 4,816,567, or obtained commercially. Monoclonal antibodies with affinities of 108 M-1, preferably 109 to 1010 M-1 or more, are preferred.

The antibody either directly or indirectly bears a suitable detectable label. Alternatively, the detectable label can be attached to a secondary antibody, e.g., goat anti-rabbit IgG, which binds the primary antibody. Frequently, the polypeptides and antibodies are labeled by joining, either covalently or noncovalently, a substance which provides a detectable signal. Suitable labels include, but are not limited to, radionuclides, enzymes, substrates, cofactors, inhibitors, fluorescent agents, chemiluminescent agents, magnetic particles and the like.

Any suitable means can be used to obtain a biological sample from a subject. A biological sample can be peripheral blood, cord blood, bone marrow, tissue sample, ascites, pleural effusions or body fluids.

Kits for determining if a subject is systemically unhealthy and at risk of developing systemic diseases and syndromes will include at least one container sized to house at least one reagent useful in determining the expression of PDPK F_{A}/GSK-3α in cell as defined herein, and printed instructions for assessing whether or not cells in a biological sample contain one or more unhealthy cells. As used herein, the term "reagent" means any compound, composition or biological agent (i.e., samples, aliquots or "doses" of cells, antibodies, etc.) useful in carrying out any method provided herein, including but not limited to antibodies for PDPK F _{A}/GSK-3a, buffers and carriers useful in isolating and preparing cells and/or membranes for analysis and treatment, buffers and carriers useful in carrying out saturation and competition binding assays, and radioactive and non-radioactive labeling compounds. The printed instructions will also include instructions for correlating the results of the tests with the unhealthy cell phenotype.

**Embodiments**

Unless otherwise indicated in the specific embodiments, all immunohistochemical analysis, immunophenotyping analysis, immunocytochemical analysis and statistical analysis followed the below methods.

Production, Identification and Characterization of Specific Anti- PDPK F_{A} /GSK-3α Antibody. The peptide QSTDATPTLTNSS, corresponding to the carboxyl terminal region from amino acids 471 to 483 of the sequence of PDPK F_{A}/GSK-3α was synthesized by peptide synthesizer (model 9050, Milligen, Bedford, MA). The cysteine residue was added to the NH2 terminus in order to facilitate coupling of the peptide to bovine serum albumin according to the procedure described by Reichlin (1980) using glutaraldehyde as the cross-linker. The antibody production has been through affinity purification and the recognition that could be blocked by the C-terminal peptide from amino acids 471-483 of PDPK F_{A}/GSK-3α to demonstrate the immunospecificity of this anti-PDPK F_{A}/GSK-3α antibody.

Immunohistochemial Analysis. Tissue sections (5 µm) of formalin-fixed, paraffin-embedded tissue containing tumor that showed the maximum extent of tumor cells were dewaxed in xylene and rehydrated in graded concentrations of ethanol. Endogenous peroxidase was blocked with 3 % hydrogen peroxide followed by bovine serum albumin blocking for 5 minutes. The slides were next incubated with anti-PDPK F_{A}/GSK-3α antibody (2 µg/mL) diluted in 0.05 M Tris buffer, pH 7.4, at 4 °C for 16 hours followed by 20-minute incubation at room temperature with super enhancer (Super SensitiveTM Non-Biotin Detection System, [BioGenex, San Ramon, CA]), and another 30-minute incubation with polymer-HRP (Super SensitiveTM) label. Immunostaining was finally developed with DAB (3-3' diaminobenzidine tetrahydrochloride). After quenching the enzyme reaction, slides were incubated in DS-enhancer (Zymed, San Francisco, CA) at room temperature for five minutes to prevent the interaction between two staining system. Then, slides were incubated with CD34,vimentin, CD68 or α-SMA antibody for one hour at room temperature. After washing, slides were incubated with anti-mouse alkaline phosphatase for 30 minutes at room temperature. BCIP/NBT solution was used for visualization of the bound antibody. Sections were counterstained with methyl green solution.

Immunocytochemical analysis. On average 1×10⁶ cells will be cytocentrifuged onto polylysine-coated slides at 700 rpm for 3 minutes at room temperature (Kubota 5200, Japan). Before staining, the cytospots will be fixed with 3.7% paraformaldehyde for 15 minutes and treated with 0.2 % triton X-100 for 90 seconds. Endogenous peroxidase will be blocked with 3 % hydrogen peroxide followed by bovine serum albumin blocking for 10 minutes. The slides will be incubated with anti-PDPK F_{A}/GSK-3α antibody (2 µg/mL) diluted in 0.05 M Tris buffer, pH 7.4, at 4°C for 16 hours followed by 20-minute incubation at room temperature with super enhancer (Super SensitiveTM Non-Biotin Detection System, [BioGenex, San Ramon, CA]), and another 30-minute incubation with polymer-HRP (Super SensitiveTM) label. Immunostaining was finally developed with DAB (3-3' diaminobenzidine tetrahydrochloride). After quenching the enzyme reaction, slides were incubated in DS-enhancer (Zymed, San Francisco, CA) at room temperature for five minutes to prevent the interaction between two staining system. Then, slides were incubated with CD34,vimentin, CD68 or α-SMA antibody for one hour at room temperature. After washing, slides were incubated with anti-mouse alkaline phosphatase for 30 minutes at room temperature. BCIP/NBT solution was used for visualization of the bound antibody. Sections were counterstained with methyl green solution.

Statistical analysis. In the statistical analyses, the samples were dichotomized as with versus without the presence of unhealthy cells. Overall survival was calculated from the date of diagnosis to the date of death or last follow-up. Disease-free survival was measured from the date of diagnosis to the date of recurrence, metastasis, death or last follow-up. The Kaplan-Meier method was used to determine the survival probability, and the log-rank test was used to compare the survival curves between groups. Two groups were compared by using chi-square, student's t and Mann-Whitney U tests, whichever appropriate. The impact of was analyzed by the Cox proportional hazards regression model. Logistic regression was used to calculate the risk of the presence of unhealthy cells in cancer family. *P<*0.05 was considered statistically significant.

The study was performed under the approved research projects and grants from National Science Council in Taiwan and approved by the informed consents and the Institution's Surveillance and Ethics Committee.

The following examples are offered to illustrate but not to limit the invention.

**Example I Aberrant expressions of PDPK F_{A}/GSK-3α in bone marrow-derived cells**

To establish the systemic role of PDPK F_{A}/GSK-3α in bone marrow-derived cell (BMDC), an independent cohort study on bone marrow of 24 leukemia patients was performed. Aberrant expressions of PDPK F_{A}/GSK-3α could be frequently detected in BMDC of leukemia patients with progressive diseases. In a cohort study of 24 cases, 14 cases were negative and 10 cases were found to be associated with aberrant expressions of PDPK F_{A}/GSK-3α. The immunophenotyping analysis with CD34 (BD Pharmingen, clone 581/CD34 specifically reactive with the class III CD34 epitope for the specific recognition of human hematopoietic stem/progenitor cells) further revealed that a rare population of CD34⁺ hematopoietic stem/progenitor cells, and CD34⁻ mesenchymal stem/progenitor cells (Moioli et al, PLoS ONE, 2008, 3:e3922) associated with very strong expressions (>3+) of PDPK F_{A}/GSK-3α and their derivatives associated with moderate to strong expressions (2+ to 3+) of PDPK F_{A}/GSK-3α (Fig. 1A) in contrast to the negative cases (Fig. 1B) could be frequently detected in bone marrow of those patients with progressive diseases. It is now clear that BMDC can be recruited and homing to the injured tissues throughout a large variety of vital organs particularly during aberrant wound healing process and chronic inflammations. The aberrant wound healing represents a significant cause of morbidity and mortality for a large portion of the population. Thus, on the bright side, BMDCs are a potential therapy for many diseases including end-stage ischemic heart disease, arterial stenosis and osteogenesis imperfecta. Conversely, on the dark side, BMDCs contribute to systemic fibrosis in many vital organs including liver, lung, kidney, intestine and bone marrow as well as to systemic fibrosis surrounding various types of tumors, suggesting that BMDCs are also involved in systemic disease development and progression in the opposite directions (Le Bousse-Kerdiles et al, Eur Cytokine Netw, 2008, 19:69-80; Lin et al, Cells Tissues Organs, 2008, 188:178-188). Due to multisubstrate/multifunctional PDPK nature, the aberrant expressions of PDPK F_{A}/GSK-3α in BMDC may represent the underlying mechanism for systemic disease development and progression. Taken together, the results from bone marrow study provide initial evidence to demonstrate the systemic role of multisubstrate/multifunctional PDPK F_{A}/GSK-3α as a systemic unhealthy signal of BMDC in the intrinsic deregulated bone marrow.

**Example II Aberrant expressions of PDPK F_{A}/GSK-3α in the stromas of various types of incurable stage I tumors**

In this embodiment, the stroma tissue specimens used for immunohistochemical analysis were obtained through a detailed retrospective review of the medical records of 367 patients with very early stage I tumors who had treatments at National Taiwan University Hospital, Taipei, Taiwan, between 1987 and 2004. Table 1 shows the stroma tissue locations, age, gender, status of survival and PDPK F_{A}/GSK-3α. Patients were observed until April, 2006.

Table 1: Patients Characteristics

**Table 1**

| Characteristics | | Case number | Percentage (%) |
|---|---|---|---|
| Origin of stroma tissue location | Breast | 17 | 4.6 |
| | Bile duct | 58 | 15.8 |
| | Colorectum | 9 | 2.5 |
| | Cervix | 18 | 4.9 |
| | Esophagus | 7 | 1.9 |
| | Stomach | 45 | 12.3 |
| | Liver | 14 | 3.8 |
| | Lung | 69 | 18.8 |
| | Oral cavity | 3 | 0.8 |
| | Ovary | 30 | 8.2 |
| | Pancreas | 42 | 11.4 |
| | Prostate | 8 | 2.2 |
| | Kidney | 47 | 12.8 |
| Gender | Male | 180 | |
| | Female | 187 | |
| Age | Range (years) | 21-89 | |
| | Mean ± SD | 58.8 ± 12.7 | |
| Status | Alive | 270 | 73.6 |
| | Death | 97 | 26.4 |
| PDPK F_{A}/GSK-3α status | Negative | 240 | 65.4 |
| | Positive | 127 | 34.6 |
| | | | |

The aberrant expressions of PDPK F_{A}/GSK-3α could be frequently detected within the stromas of various types of stage I tumors with poor prognosis even after curative resections. In a cohort study of 367 stage I tumors, 240 cases were negative and 127 cases were associated with aberrant expressions of PDPK F_{A}/GSK-3α. Patients if associated with aberrant expression of this signaling molecule within tumor stromas tend to develop poor outcome (Fig. 2). Immunophenotyping analysis with CD34, CD68, vimentin and α-smooth muscle actin (α-SMA) further revealed that a rare population of CD34⁺/vimentin⁻ hematopoietic stem/progenitor cells, CD34⁺/vimentin⁺fibrocytes and CD34⁻/vimentin⁺ mesenchymal stem/progenitor cells (Bucala, Fibrocytes, World Scientific Pub Co Inc, 2007:1-18; Moioli et al, PLoS ONE, 2008, 3:e3922) associated with very strong expressions (>3+) of PDPK F_{A}/GSK-3α (Fig. 3A and C) essentially as described in Example I along with a relatively large population of CD68⁺ macrophages (Fig. 3E), vimentin⁺ fibroblasts and mesenchymal cells (Fig. 3C) and α-SMA⁺ myofibroblasts (Fig. 3G) associated with moderate to strong expressions (2+ to 3+) of PDPK F_{A} /GSK-3α in contrast to the negative cases with good outcome (see Figs. 3B, D, F and H) could be detected predominantly within the stromas of incurable stage I tumors with poor prognosis even after curative resections. Regardless of the etiology of the stromas of breast, bile duct, colorectum, cervix, esophagus, stomach, liver, lung, oral cavity, ovary, pancreas, prostate and kidney, if associated with aberrant expressions of PDPK F_{A}/GSK-3α, the stage I patients tend to develop systemic fibroses, infections, obstructions, emboli, ulcer, UGI bleeding and multiple organ failures. The Cox regression analysis further revealed that the positive stage I patients had more than 6-fold the risk of developing systemic diseases and syndromes compared to the negative stage I patients (hazard ratio 6.258, 95%Cl 4.125-9.493 for disease-free survival, *P* <0.001). Although Hanahan and Weinberg (Cell, 2000, 100:57-70) had enumerated the hallmarks of cancer, the developed therapies still failed approximately half populations of the patients. The recent studies have challenged that the incurable early-stage tumors are merely the end products of the systemic bone-marrow diseases and represent only a small population of systemic chronic diseases. The intimate relationship between bone marrow and tumor stroma is now more widely acknowledged. There is increasing evidence that bone marrow contributes to stromal lineages in a variety of situations from aberrant wound healing to end-stage systemic fibrotic diseases and/or neoplasia and BMDC is a cell type that is recruited in large numbers to the stroma of fibrotic tissues and/or developing tumors (Direkze and Alison, Hematol Oncol, 2006, 24: 189-195; Bellini and Mattoli, Lab Invest, 2007, 87:858-870; Karnoub et al, Nature, 2007, 449:557-563; Lin et al, Cells Tissues Organs, 2008, 188: 178-188). It is now clear that in addition to local resident cells and EMT process, BMDCs represent the original resource contributing to systemic tissue homeostasis from aberrant wound healing to end-stage systemic fibrotic disease and/or neoplasia. The intrinsic defect in bone marrow stem and progenitor cells and their derivatives such as stromal and inflammatory cells may represent the major cause for aberrant wound healing, chronic inflammation and related systemic diseases and syndromes as described above. Thus, the disease entities of stage I tumors if incurable with local excisions, are no longer tumor-specific diseases; they are host-specific, bone marrow-systemic diseases at the onset. Taken together, the results provide further evidences to demonstrate the systemic role of PDPK F_{A}/GSK-3α as a systemic unhealthy signal of BMDC in intrinsic deregulated bone marrow. The combined results establish PDPK F_{A}/GSK-3α as a systemic signal for very early prediction of systemic aberrant wound healing and the systemically unhealthy status of a subject regardless of the etiological origin of the beyond curable disease. The combined results support the current notion that the stromas of developing tumors and/or fibrotic tissues are often accompanied by the recruitment of a variety of stromal cells closely resembling aberrant wound healing which entails the constant deposition of growth factors, cytokines, matrix-remodeling protein and connective tissue elements to cause multiple organ failures. The combined results are also in agreement with the notion that the BMDC may represent the most primitive uncommitted cancer stem cell if recruited to a chronically inflamed tissue as proposed by Takaishi et al. (J Clin Oncol, 2008, 26: 2876-2882). It is noted that, on some occasions, we did observe a rare population of CD44⁺ cells characteristic of cancer stem cells associated with very strong expressions of PDPK F_{A}/GSK-3α in the stromas of incurable stage I tumors (data not further illustrated). By using PDPK F_{A} /GSK-3α and the BMDC system presented in Examples I and II, the universally applicable molecule and cell expression profiles for determining a subject's risk of developing systemic diseases and syndromes can be developed. The present invention establishes PDPK F_{A}/GSK-3α in BMDC as a very reliable predictor of a subject's likelihood or susceptibility to develop a beyond curable systemic disease regardless of its etiological origin but due to systemic seeding during aberrant wound healing.

**Example III Aberrant expressions of PDPK F_{A}/GSK-3α in inflammatory fibrotic tissues**

In accordance with the notion as established above, the aberrant expressions of PDPK F_{A}/GSK-3α indeed could be frequently detected in inflammatory fibrotic tissues. In an independent cohort study of 35 squamous hyperplastic tissues, comprising 26 cases without fibrosis and 9 cases with fibrosis, aberrant expression of PDPK F_{A}/GSK-3α in BMDC as shown in Figs. 1 and 3 were detected predominantly in hyperplastic tissues with fibrosis; 7 of 9 were found to be positive with PDPK F_{A} /GSK-3α. Conversely, 18 of 26 cases of fibrosis-negative tissues were found to be PDPK F_{A}/GSK-3α-negative (Chi-square test, *P*=0.022). Immunophenotyping analysis with CD34, CD68, vimentin and α-SMA further revealed that a rare population of CD34⁺/vimentin⁻ hematopoietic stem/progenitor cells, CD34⁺/vimentin⁺fibrocytes and CD34⁻/vimentin⁺ mesenchymal stem/progenitor cells associated with very strong expression (>3+) of PDPK F_{A}/GSK-3α along with a large population of CD68⁺ macrophages, vimentin⁺ fibroblasts and mesenchymal cells and α-SMA⁺ myofibroblasts associated with moderate to strong expressions (2+ to 3+) of PDPK F_{A}/GSK-3α could be frequently detected in inflammatory fibrotic tissues in contrast to the negative cases without fibrosis essentially described in Examples I and II (not further illustrated). There is increasing evidence that BMDCs as shown in Fig. 1 contribute to the new population of stem/progenitor cells and derivatives such as vimentin⁺ fibroblasts, α-SMA⁺ myofibroblasts and CD68⁺ macrophages at the tissue site particularly during aberrant wound healing in inflammatory fibrotic tissues. Fibrotic diseases are an important cause of morbidity and mortality worldwide. They occur in a large variety of vital organs and the process of systemic fibrosis is common to all tissues. The different BMDC types involved in this process may work together to stimulate the deposition of connective tissue elements that progressively destroy the normal tissue architecture with the loss of organ functions leading to death (Dunsmore and Shapiro, J Clin Invest, 2004, 113:180-182; Bellini and Mattoli, Lab Invest, 2007, 87:858-870; Bucala, Fibrocytes, World Scientific Pub Co Inc, 2007:1-18; Le Bousse-Kerdiles et al, Eur Cytokine Netw, 2008, 19:69-80; Lin et al, Cells Tissues Organs, 2008, 188:178-188). Taken together, the results provide initial evidence to demonstrate that aberrant expressions of PDPK F_{A}/GSK-3α in BMDC were associated with fibrosis characteristic of aberrant wound healing and chronic inflammations as described above. All the results as shown in Examples I-III taken together provide further evidence to demonstrate the systemic role of PDPK F_{A}/GSK-3α as a systemic unhealthy signal of BMDC in the intrinsic deregulated bone marrow.

**Example IV Aberrant expressions of PDPK F_{A}/GSK-3α in peripheral blood of systemically unhealthy children from cancer family or suffering from repeated cycles of inflammations**

There is increasing evidence that CD34⁺/vimentin⁺ fibrocytes, the bone marrow-derived mesenchymal progenitor in peripheral blood contribute to the new population of fibroblasts and myofibroblasts during aberrant wound healing and inflammatory fibrotic processes (Bucala et al, Mol Med, 1994, 1:71-81; Schmidt et al, J Immunol, 2003, 171:380-389; Bellini and Mattoli, Lab Invest, 2007, 87:858-870; Bucala, Fibrocytes, World Scientific Pub Co Inc, 2007:1-18; Lin et al, Cells Tissues Organs, 2008, 188: 178-188). In agreement with this notion, CD34⁺ fibrocyte-like cell associated with strong expression of PDPK F_{A}/GSK-3α could be detected in peripheral blood of all the tested 3 children from 3 different families suffering from repeated cycles of inflammation (Fig. 4). Such type of unhealthy cell was associated with human hypertrophic scars, nephrogenic systemic fibrosis, atherosclerotic lesions and pulmonary fibrosis mainly originated from BMDC. Taken together with Examples I-III, the results provide comprehensive evidences to demonstrate the systemic role of PDPK F_{A}/GSK-3α as a very early unhealthy signal of BMDC in the intrinsic deregulated bone marrow.

In accordance with this concept, of 33 normal individuals studied (13 male and 20 female) with ages ranging from 10 to 65 years (mean±SD, 42.5±13.1), the cancer family members tend to have unhealthy cell detected in their blood (*P*=0.001). The logistic regression revealed that cancer family members had more than 13-fold the risk of the presence of unhealthy cell compared to non-cancer family people (OR for the presence of unhealthy cell 13.333, 95% Cl 2.454-72.452, *P*=0.003; Table 2). The results further demonstrate that the cancer family members tend to have high risk of developing intrinsic defect in bone marrow associated with unhealthy BMDCs highly expressing PDPK F_{A}/GSK-3α, which provides a method of predicting if a subject is systemically unhealthy and at risk of developing systemic diseases and syndromes as established in Examples I-IV.

Table 2: The risk of the presence of unhealthy cell in cancer families

**Table 2**

| | Correlation^{†} | | | Logistic regression^{‡} | | |
|---|---|---|---|---|---|---|
| | The presence of unhealthy cell | | *P** | OR for the presence of unhealthy cell | 95 % Cl | *P** |
| | - | + | | | | |
| Age | 44.4±1 | 41.1±1 | NS | | | |
| | 3.4 | 3.1 | | | | |
| Gender | | | | | | |
| Male | 5 | 8 | | | | |
| Female | 9 | 11 | NS | | | |
| Group | | | | | | |
| Non-cancer family | 10 | 3 | | 1 | | |
| Cancer family | 4 | 16 | 0.001 | 13.333 | 2.454-72.452 | 0.003 |
| | | | | | | |

Abbreviations: OR, odds ratio; 95% Cl, 95% confidence interval

**P*<0.05 was considered statistically significant (^{†}chi-square test and ^{‡} logistic regression)

**Example V Aberrant expression of PDPK F_{A}/GSK-3α in umbilical cord blood of systemically unhealthy child suffering from Kawasaki syndrome**

In agreement with the results presented above, such type of unhealthy BMDC also could be detected in the umbilical cord blood of systemically unhealthy children suffering from immune system disorder characteristic of Kawasaki syndrome (Fig. 5). The results provide further evidence to demonstrate the systemic role of PDPK F_{A}/GSK-3α as a very early unhealthy signaling molecule associated with cord blood and bone marrow stem/progenitor cells. All the results combined, this invention has established PDPK F_{A}/GSK-3α as a systemic unhealthy signal of BMDC throughout the life span of a subject suffering from systemic diseases starting from a very early symptoms-developing stage throughout a very final syndromes-developed stage.

**Example Vl Aberrant expression of PDPK F_{A}/GSK-3α in nonhealing wound: ulcer**

Aberrant expressions of PDPK F_{A}/GSK-3α could be frequently detected in ulcerative stromas. In an independent cohort study of 39 oral tissues with ulcer, 34 of 39 cases were associated with aberrant expressions of PDPK F_{A}/GSK-3α in a hierarchical population of BMDCs comprising a rare population of CD34⁺/vimentin⁻ hematopoietic stem/progenitor cells and CD34⁻/vimentin⁺ mesenchymal stem/progenitor cells associated with very strong expressions (>3+) of PDPK F_{A}/GSK-3α and their derivatives associated with moderate to strong expressions (2+ to 3+) of PDPK F_{A} /GSK-3α within stroma essentially as described above (not further illustrated). The results provide further evidence to demonstrate the systemic role of PDPK F_{A}/GSK-3α as an impaired wound healing-associated unhealthy signal of BMDC associated with intrinsic defects in bone marrow.

In summary, the present invention has established PDPK F_{A}/GSK-3α as a very early unhealthy signal and also a final lethal signal in bone marrow and cord blood. PDPK F_{A}/GSK-3α therefore represents a systemic unhealthy signal of intrinsic deregulation of BMDC. Thus, the present invention provides a detection system to diagnose and predict the health and disease status of bone marrow and cord blood prior to transplations. The unhealthy BMDC and PDPK F_{A}/GSK-3α presented in this invention represent a very early molecular and cellular system to predict the health and disease statue throughout the life span of a subject associated with systemic bone marrow problems. 1. In terms of the above, the present invention provides a method of predicting if a subject is systemically unhealthy and at risk of developing systemic diseases and syndromes by using PDPK F_{A}/GSK-3α as a diagnostic marker.

**Reference**

Bellini A, Mattoli S (2007) The role of the fibrocyte, a bone marrow-derived mesenchymal progenitor, in reactive and reparative fibroses. Lab Invest87: 858-870

Bingle L, Brown NJ, Lewis CE (2002) The role of tumour-associated macrophages in tumour progression: implications for new anticancer therapies. J Pathol196: 254-265

Biswas SK, Sica A, Lewis CE (2008) Plasticity of macrophage function during tumor progression: regulation by distinct molecular mechanisms. J Immunol180: 2011-2017

Bucala R (2007) Fibrocytes. Fibrocytes, World Scientific Pub Co Inc. 1-18

Bucala R, Spiegel LA, Chesney J, Hogan M, Cerami A (1994) Circulating fibrocytes define a new leukocyte subpopulation that mediates tissue repair. Mol Med1: 71-81

Chung YC, Chang KJ, Yang CC, Lai MT, Hsu CP, Hsueh SF, Peng CC, Fu HH, Chang YF, Yang SD (2002) Association of proline-directed protein kinase FA with tumorigenesis, invasion, and poor prognosis of human colon carcinoma. Cancer95: 1840-1847

Condeelis J, Pollard JW (2006) Macrophages: obligate partners for tumor cell migration, invasion, and metastasis. Cel/124: 263-266

De Wever O, Mareel M (2003) Role of tissue stroma in cancer cell invasion. J Pathol200: 429-447

Direkze NC, Alison MR (2006) Bone marrow and tumour stroma: an intimate relationship. Hematol Oncol24: 189-195

Dunsmore SE, Shapiro SD (2004) The bone marrow leaves its scar: new concepts in pulmonary fibrosis. J Clin Invest113: 180-182

Fu HH, Yang SD (2004) Suppression of proline-directed protein kinase F(A) inhibits the malignant growth of human pancreatic ductal adenocarcinoma. Anticancer Res24: 1489-1494

Hsu CP, Hsueh SF, Yang CC, Yang SD (2000) Suppression of proline-directed protein kinase F(A) expression inhibits the growth of human chronic myeloid leukaemia cells. BrJ Cancer82: 1480-1484

Hsu CP, Yang CC, Hsueh SF, Peng CC, Fu HH, Yang SD (2001) Suppression of proline-directed protein kinase F(A) potentiates apoptotic induction and greatly enhances chemosensitivity in human acute lymphoblastic leukemia cells. Cancer92: 1753-1758

Hsu CP, Yang CC, Yang SD (2000) Suppression of proline-directed protein kinase F(A) expression potentiates erythroid differentiation of human myeloid leukemia cells. Cancer89: 1004-1011

Hsu CP, Yang CC, Yang SD (2001) Suppression of proline-directed protein kinase F(A) systemically inhibits the growth of human acute leukemia cells. Int J Cancer91: 650-653

Hsueh SF, Lai MT, Yang CC, Chung YC, Hsu CP, Peng CC, Fu HH, Cheng YM, Chang KJ, Yang SD (2002) Association of overexpressed proline-directed protein kinase F(A) with chemoresistance, invasion, and recurrence in patients with bladder carcinoma. Cancer95: 775-783

Karnoub AE, Dash AB, Vo AP, Sullivan A, Brooks MW, Bell GW, Richardson AL, Polyak K, Tubo R, Weinberg RA (2007) Mesenchymal stem cells within tumour stroma promote breast cancer metastasis. Nature 449: 557-563

Kisseleva T, Brenner DA (2008) Mechanisms of fibrogenesis. Exp Biol Med233: 109-122

Le Bousse-Kerdiles MC, Martyre MC, Samson M (2008) Cellular and molecular mechanisms underlying bone marrow and liver fibrosis: a review. Eur Cytokine Netw19: 69-80

Lin WR, Brittan M, Alison MR (2008) The role of bone marrow-derived cells in fibrosis. Cells Tissues Organs188: 178-188

Mishra PJ, Glod JW, Banerjee D (2009) Mesenchymal stem cells: flip side of the coin. Cancer Res69: 1255-1258

Moioli EK, Clark PA, Chen M, Dennis JE, Erickson HP, Gerson SL, Mao JJ (2008) Synergistic actions of hematopoietic and mesenchymal stem/progenitor cells in vascularizing bioengineered tissues. PLoS ONE3: e3922

Schafer M, Werner S (2008) Cancer as an overhealing wound: an old hypothesis revisited. Nat Rev Mol Cell Biol9: 628-638

Schmidt M, Sun G, Stacey MA, Mori L, Mattoli S (2003) Identification of circulating fibrocytes as precursors of bronchial myofibroblasts in asthma. J Immunol171: 380-389

Valtieri M, Sorrentino A (2008) The mesenchymal stromal cell contribution to homeostasis. J Cell Physiol217: 296-300

Vandenheede JR, Yang SD, Goris J, Merlevede W (1980) ATP x Mg-dependent protein phosphatase from rabbit skeletal muscle. II. Purification of the activating factor and its characterization as a bifunctional protein also displaying synthase kinase activity. J Biol Chem255: 11768-11774

Woodgett JR (1990) Molecular cloning and expression of glycogen synthase kinase-3/factor A. EMBO J9: 2431-2438

Wynn TA (2008) Cellular and molecular mechanisms of fibrosis. J Pathol 214: 199-210

Yang CC, Hsu CP, Yang SD (2000) Antisense suppression of proline-directed protein kinase FA enhances chemosensitivity in human prostate cancer cells. Clin Cancer Res6: 1024-1030

Yang SD (2004) Proline-directed protein kinase FA as a potential target for diagnosis and therapy of human cancers. Curr Cancer Drug Targets4: 591-596

Yang SD, Vandenheede JR, Goris J, Merlevede W (1980) ATP x Mg-dependent protein phosphatase from rabbit skeletal muscle. I. Purification of the enzyme and its regulation by the interaction with an activating protein factor. J Biol Chem255: 11759-11767

Yin T, Li L (2006) The stem cell niches in bone. J Clin Invest116: 1195-1201

## Claims

1. A method for detecting the presence of a cellular expression profile in a subject indicative of unhealthy cell, which method comprises: obtaining a biological sample from said subject; and determining the expression of PDPK F_{A}/GSK-3α in cell in said sample; wherein an increased expression level of PDPK F_{A} /GSK-3α in a cell in comparison to a normal cell indicates the presence of unhealthy cell.

2. The method of claim 1, wherein said biological sample is bone marrow, cord blood, peripheral blood, tissue sample, ascites, pleural effusions or body fluids.

3. The method of claim 1, wherein said expression of PDPK F_{A}/GSK-3α is determined by assessing PDPK F_{A}/GSK-3α protein, mRNA, DNA or activity level.

4. The method of Claim 1, wherein the cell is a stem/progenitor cell or derivative, selected from a group consisting of bone marrow-derived cell, mesenchymal stem/progenitor cell, hematopoietic stem/progenitor cell, cancer stem cell, fibrocyte, macrophage, fibroblast, myofibroblast, mesenchymal cell and the like.

5. A method for predicting if a subject is systemically unhealthy and at risk of developing systemic diseases and syndromes, which method comprises:
obtaining a biological sample from said subject; and determining the expression of PDPK F_{A}/GSK-3α in said sample; wherein an increased expression level of PDPK F_{A}/GSK-3α in comparison to a normal cell predicts if a subject is systemically unhealthy and at risk of developing systemic diseases and syndromes.

6. The method of claim 5, wherein said biological sample is bone marrow, cord blood, peripheral blood, tissue sample, ascites, pleural effusions or body fluids.

7. The method of claim 5, wherein said expression of PDPK F_{A}/GSK-3α is determined by assessing PDPK F_{A}/GSK-3α protein, mRNA, DNA or activity level.

8. The method of Claim 5, wherein the cell is a stem/progenitor cell or derivative, selected from a group consisting of bone marrow-derived cell, mesenchymal stem/progenitor cell, hematopoietic stem/progenitor cell, fibrocyte, macrophage, fibroblast, myofibroblast and the like.

9. A diagnostic kit for identifying and detecting an unhealthy cell in a biological sample, comprising one or a plurality of reagents to PDPK F_{A}/GSK-3α; and optionally, instructions for use.
